(19)

Europäisches
Patentamt
European
Patent Office
Office européen
des brevets

(11) **EP 3 922 223 A1**

(12) **EUROPEAN PATENT APPLICATION**
published in accordance with Art. 153(4) EPC

(43) Date of publication:
**15.12.2021 Bulletin 2021/50**

(21) Application number: **19914567.3**

(22) Date of filing: **11.02.2019**

(51) Int Cl.:
*A61F 5/37* (2006.01)      *A61H 1/02* (2006.01)
*A61G 15/12* (2006.01)

(86) International application number:
**PCT/RU2019/000081**

(87) International publication number:
**WO 2020/162781 (13.08.2020 Gazette 2020/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **04.02.2019   RU 2019103045**

(71) Applicants:
• **Belykh, Yury Aleksandrovich
Moskovskaya obl., 141551 (RU)**

• **Ivanov, Aleksandr Yurievich
Kaluzhskaya obl., 249030 (RU)**

(72) Inventors:
• **Belykh, Yury Aleksandrovich
Moskovskaya obl., 141551 (RU)**
• **Ivanov, Aleksandr Yurievich
Kaluzhskaya obl., 249030 (RU)**

(74) Representative: **Vitina, Maruta et al
Agency TRIA ROBIT
P.O. Box 22
1010 Riga (LV)**

(54) **ARM SUPPORT DEVICE**

(57)      SUBSTANCE: invention relates to medical devices designed to support a hand with provision of unloading and compensation / restoration of a weakened hand muscle as a result of diseases or injuries. In particular, the device can be used in hospitals or rehabilitation clinics, incl. when conducting exercises to restore function / movement of the hand in patients suffering from neurological and orthopedic diseases, which manifest themselves in the form of proximal weakening of the upper limb. The device can also be used at home to perform everyday tasks that facilitate arm movement in people with reduced arm function, muscular dystrophy of the upper limb.

The arm support device includes a support stand with a housing attached to it and rotatable about the vertical axis of the support stand; a pivot frame fixed in the body with the possibility of rotation about the vertical axis of the support post in the horizontal plane and angular movement relative to the horizontal axis of the frame attachment to the body in the vertical plane, while the frame includes an upper element in the form of a first lever, a lower element in the form of a rod, which are hinged connected to the body and to the side element to form a parallelogram; a support element for the hand, functionally connected to the pivot frame through the second lever, wherein the second lever is rotatable about the vertical axis of the connection of the second lever with the rotary frame, and the support element for the hand is rotatable about the vertical and horizontal axes, by means of which connecting the support member to the second arm; tensioner unit, fixed in the housing, including an elastic element connected to the pivot frame with the possibility of adjusting the tension force of the elastic element to compensate for the weight of the hand. A distinctive feature of the invention is the implementation of the first arm of the pivot frame with an arm that implements the function of a drive bracket, located on the opposite side from the horizontal axis of attachment of the frame to the base, as well as the location of the tensioner block on the opposite side of the pivot frame relative to the axis of the support post, while serving as an elastic element The tensioner unit uses at least one spring of constant force, the free end of which is connected to the drive bracket with the possibility of adjusting the tension of the spring.

# Description

The technical field to which the invention relates

SUBSTANCE: invention relates to medical devices designed to support a hand with provision of unloading and compensation / restoration of a weakened hand muscle as a result of diseases or injuries. In particular, the device can be used in hospitals or rehabilitation clinics, incl. when conducting exercises to restore function / movement of the hand in patients suffering from neurological and orthopedic diseases, which manifest themselves in the form of proximal weakening of the upper limb. The device can also be used at home to perform everyday tasks that facilitate arm movement in people with reduced arm function, muscular dystrophy of the upper limb. The claimed invention relates directly to devices for dynamic support / arm support, enabling the ability to move the arm in the horizontal and vertical planes with gravity compensation to support the arm.

## State of the art

Various rehabilitation medical devices are known for restoring the functions of the musculoskeletal system of the limbs, providing medical rehabilitation assistance and restoring / maintaining the activity of the upper limbs, incl. in the elderly and disabled.

Three main groups of hand support devices are known in the art.

The first group consists of devices with an emphasis without vertical movement of the arm Original MAS (Mobile Arm Support) by Jaeco Orthopedic (Available as of 01.02.2019 on the Internet at https://www.neurorehabdirectory.com/rehab-products/original-mobile-arm-support /), in which the position of the arm is set and fixed at a certain height and is hinged at the fulcrum. The devices are predominantly attached to a movable object, such as a wheelchair, or to a stationary object. Typically, such devices are designed for specific activities, allow only simple movements (flexion / extension) in the elbow and wrist joints without involving the muscles of the proximal shoulder, and do not allow shoulder abduction in the frontal plane; the rigid articulated support does not allow full natural arm movements.

The second group of devices consists of arm support systems with the addition of counterweights, which passively compensate for the influence of the arm's gravity, balance the arm's weight in any range of its movements, for example, the Mobility Arm by Nitzbon (Available as of 01.02.2019 on the Internet at https: //www.neurorehabdirectory.com/rehab-products/mobility-arm/). The disadvantage of such systems is the single-lever design, characterized by a constant ratio of the shoulders, on which the weight of the arm and counterweights is applied. The arm, placed in the cradle, can move up and down and rotate around the axis of the levers and arm suspension. When the hand is moved from the axis and towards the axis of the balance bar, the shoulder of the application of the hand weight changes and the balance of the system is disturbed, which significantly reduces the efficiency of using such systems.

The third group of devices consists of arm support systems that use mechanisms in their design that change the value of the balancing moment by changing the shoulder of the force application.

In particular, an arm support device is known, in which a gas spring is used instead of a load, and the change in the shoulder of the force application is implemented by changing the value of the balancing moment, for example, Armon Edero (Available as of 01.02.2019 on the Internet at https://www.neurorehabdirectory.com/rehab-products/armon-edero/ or http://heamedical.se/produkt/armon-edero/), or the solution presented in patent publication CN106901945, or the solution presented in the utility model patent RU166673.

Publication CN106901945 discloses a device secured to a fixed base pivotally connected to a first arm, allowing rotation about the axis of the first arm; the other end of the first arm is connected by a round pin (rotatable around it) to the end of the arcuate connecting plate; the lower end of the arcuate plate rotates simultaneously with its upper end, on which a second lever is fixed through the axial connection, which can move relative to the axial connection. A gas spring is connected to one end of the second lever, and a universal joint is connected to the other end, through which a mechanical connection is made to the support platform for the forearm of the hand. The resistance force of the gas spring can be changed by changing the point of its attachment to the connecting plate using a special mechanism.

In publication RU166673, a mechanical device for supporting a hand is presented, comprising a cradle for the hand; a rod, the distal end of which is pivotally connected to the cradle to ensure the mobility of the cradle in the vertical plane; an arm pivotally connected to the proximal end of the bar to provide vertical mobility of the bar under the weight of the hand; a horizontal adjustment bar on which the bracket is fixed; a gas compression spring, the distal end of which is connected to the rod through a movable hinge located on the rod with the ability to move along it and fix it on it, and the proximal end is pivotally connected to the adjusting bar with the ability to move along it and fix it on it, and an additional bar, hinged connected to the adjusting bar to ensure its mobility in the horizontal plane, and the additional bar is made to communicate with the fixed support.

These solutions compare favorably with the systems of the first group in ease of use, compactness and capabilities for movement in the horizontal plane. The main disadvantage of such systems is the property of a gas spring, which has a "step" in force at the moment of transition from static friction to sliding friction from the initial position. This deprives the systems of the smoothness inherent in mechanical systems. In addition, the possibility of vertical movement of the hand is limited by the stroke of the gas spring, which is associated both with the dimensions of the gas spring itself and with the overall dimensions of the device, the weight and inertia of the masses being moved. In addition, the device uses a first class lever, which increases the size of the lever by the length of the arm. In addition, the device does not allow for vertical adjustment of the lower position of the cradle, which significantly limits its functionality. Such systems are used only as an assistive support when performing manipulations with a small range of arm movements in a sitting position at the table, and are not suitable for restoring full range of motion in the shoulder joint.

The most promising in the third group are systems using a hinge mechanism in the form of a parallelogram with a spring element. Either elastic cables or coil springs are used as a spring element. These systems are compact and at the same time scalable, they can have practically any ranges of movement and degrees of freedom. Adjustment of the amount of compensation for the weight of the hand is carried out by adjusting the tension of the spring, as well as by changing the arm of the application of the traction force of the springs.

In particular, a passive dynamic armrest TOP / HELP (Focal Meditech) is known (Available as of 01.02.2019 on the Internet at the link https://www.researchgate.net/publication/255959271_An_overview_and_categorization_of _dynamic_arm_supports_for_people_with_decreased) with the ability to move in height and rotate around the axis of the rack. At one of the ends of the base, there is an attachment point for the first lever made in the form of a parallelogram with elastic tensioners between two ribs, which create a force to support the hand. The tension is adjusted by stretching or compressing the tensioners by moving and fixing the position of the tensioners on one of the edges of the parallelogram. When the position of the arm changes in height, the first lever (parallelogram) changes its geometric shape with a change in the distance between the fastening points of the tensioners on the levers, and, accordingly, a change in the force applied to the ribs of the parallelogram created by the tensioners. On the other hand, to the first lever (parallelogram) a system of four levers is connected through a hinge, also connected to each other by means of hinges that allow the levers to rotate in a horizontal plane. On the last, farthest from the rack, the lever is a hinge with a cradle for the forearm, which can move (swing) relative to the point of connection with the lever in a small range limited by the design characteristics of the hinge, lodgment and lever.

It is also known a device for mobile support of the SaeboMAS hand (Available as of 01.02.2019 on the Internet at the link http://www.saebo.su/products/saebomas/), described in the application US2013087154 (closest prior art). The device includes a swing frame fixed to a support post. The pivot frame includes upper and lower elongated elements pivotally connected to the side inner and outer elements to form a parallelogram, in which the outer side element is movable vertically (up and down) relative to the inner side element while maintaining the parallel arrangement of the said elements. The device also contains a support element for the hand (or cradle), functionally connected to the pivot frame; and a tensioner mechanism that includes an elastic element (spring), the tensioner mechanism is configured to selectively adjust the amount of force required to move the support element relative to the inner side element, and the elastic element is located inside one of the elongated elements and exerts a tensile force parallel to it longitudinal axis.

The disadvantage of these systems is the nonlinearity of the stiffness of the springs and the change in the arm of the application of force during vertical movement, and therefore, when lifting the arm, the compensating force can fluctuate in the range of more than 50%. This imposes restrictions on the range of vertical movement of the arm, and, as a consequence, on the patient's ability to carry out the necessary manipulations or training exercises. As a result, dead zones appear in the range of motion of the hand, in which natural movement is impossible. In the process of restoring the motor function of the hand, the presence of "dead zones" contributes to the formation of pathological motor patterns that compensate for the lack of range of motion. For example, restriction of movement during shoulder abduction due to the deltoid muscles leads to a compensatory increase in the tone of the upper portion of the trapezius muscle and the deep muscles of the neck.

**Disclosure of invention**

The technical problem solved by the invention is the development of a device for dynamic support of the hand, characterized by the absence of the disadvantages inherent in the above analogs and the prototype.

The technical result is manifested in the provision of a physiological range of motion of the hand with the maximum possible constant effort to support the hand in the entire range of motion with a minimum inertia of the device.

The technical result is achieved by the fact that in the device for supporting the hand, including a support post with a body fixed thereon with the ability to rotate around the vertical axis of the support post; a pivot frame fixed in the body with the possibility of rotation around a vertical axis of the support post in the horizontal plane and with the ability of an angular movement relative to a horizontal axis of the pivot frame attachment to the body in a vertical plane, while the pivot frame includes upper element in the form of a first lever, a lower element in the form of a rod, which are hinged connected to the body and to a side element forming a parallelogram; a support element for a hand, functionally connected to the pivot frame through a second lever, wherein the second lever is rotatable relative to the vertical axis of the connection of the second lever with the pivot frame, and the support element for the hand is rotatable relative to the vertical and horizontal axes, by means of which connecting the support member to the second lever; a tensioner unit; the tensioner unit is fixed in the body, including an elastic element connected to the pivot frame with the possibility of adjusting a tension force of the elastic element to compensate for the weight of a hand; According to the invention, the first lever of the pivot frame is made with a shoulder that performs the function of a drive bracket, the tensioner unit is located on the opposite side from the horizontal axis of the pivot frame attachment to the base, the tensioner unit is located on the opposite side of the pivot frame relative to the axis of the support post, while the tensioner unit is an elastic element used at least one constant force spring, the free end of said constant force spring is connected to the drive bracket with the possibility of adjusting the tension of the spring.

The body can be made in the form of two flat metal plates, between which spring elements are fixed on the horizontal axes.

Two springs of the KKF series with a total force of at least 120N can be used as constant force springs.

The drive bracket is preferably made of steel and the first and second levers are made of aluminum alloys.

In one of the embodiments of the invention, to adjust the tensioning force of the elastic element, the tensioner unit is provided with a screw located in the drive bracket, made with a handle and an element for securing the end of the elastic element with the ability to move along the screw and fix in a selected position, while the drive bracket is provided with a corresponding groove for placement of the screw with the said element, as well as a scale for controlling the compensating force. The length of the groove on the drive bracket with the screw located therein is selected to ensure a threefold change in the compensating force. The initial position of the groove with the screw placed therein is selected at a distance of no more than 65 mm from the horizontal axis of the first lever attachment to the body, and the final position is not less than 205 mm.

The first lever in the general case can be made with a length from 350 mm to 450 mm, while the tensioner block includes two elastic elements in the form of constant force springs with a total force of up to 12 kg.

The movement of the frame relative to the horizontal axis of fastening the frame to the body can be limited to an angle of rotation ± 30 ° from the horizontal position by means of a limiter fixed in the body.

The side element of the frame can be made in the form of a bracket on which the vertical axis of the connection of the second lever with the pivot frame is fixed.

The first and second levers are made with a length that allows the hand support element to be brought to the support post.

The arm support is equipped with removable straps.

In a particular embodiment of the invention, the vertical axes and the horizontal axes of the first lever are mounted in angular contact bearings, and the axes at the ends having a threaded section for adjusting the size of clearances in the angular contact bearings.

The second lever is preferably made with a closed cross-sectional profile.

The support post can be mounted on a horizontal working surface or on a movable base.

The drive bracket is preferably designed as an extension of the first lever. In one embodiment of the invention, the first arm and the drive arm are made of two different parts connected together by known fastening methods.

The support post can be made with the ability to change the height with the fixation of the selected position.

The claimed design allows, with minimal resistance and inertia, to make natural hand movements in full, actively involving the shoulder and elbow joints in the movement. Accurate adjustment of the compensating force of the spring mechanism allows leveling the weight of the limb, opening up the possibility of active movements even with minimal preservation of muscle strength, which allows achieving the best result in the process of rehabilitation treatment of patients with motor disorders of the upper limbs, minimizing the pathological effects of the disease on the patient. Thus, the device provides a minimum change in the compensating force from the weight of the hand at the extreme upper point and the extreme lower point, which is manifested in the movement of the arm up and down without additional resistance. In this case, the adjustment of the spring force is possible by the patient himself.

The technical result is achieved through the use of a scheme of balancing "swing", in which the compensating force is formed by means of constant force springs acting from the opposite hand of the side relative to the axis of rotation (axis of the post) and in the opposite direction. Adjustment of the value of the compensating force is carried out by changing the arm of the application of the force of the constant force spring. The balancing "swing" scheme has an advantage over systems in which the spring element is located on the same side as the arm (cantilever scheme), due to the fact that in the "swing" scheme part of the weight of the device itself is compensated by the weight of the opposite part " swing ". In cantilever systems, the spring must compensate for the weight of the hand and the weight of the device, which increases the unevenness of the compensating force, since the weight of the hand is only a part of the compensated force, and fluctuations in the magnitude of the compensating force due to changes in the length of the spring and the arm of application of force during vertical movement account for on the weight of the entire system "hand" plus "device". For the patient, this change in effort is more noticeable. In systems with balancing "swing" part of the weight of the device is compensated by the weight of the opposite part. To improve balancing, part of the device on the arm side can be made of aluminum alloys, and the part of the device on the side of constant force springs - from ordinary carbon steels. Another advantage is the use of constant force springs as opposed to elastic elements and traditional springs of constant stiffness. In springs of constant stiffness, the force varies in proportion to the length of extension, which leads to uneven compensating force during vertical movement of the arm. Constant force springs are devoid of this drawback.

An additional advantage of this technical solution is the ability to adjust the height of the device, which makes it possible to perform exercises in a sitting and standing position, which contributes to the development of coordination and balance of the patient in conditions as close to natural as possible.

Another additional advantage of this technical solution is the ability to scale the device, which is not provided in cantilever systems in which the weight of the device, the overall dimensions of the spring elements, the height of movement, the force developed by the springs are interconnected, and when scaling the device, significant restrictions arise, in particular , the increased rigidity of the spring leads to an increase in the dimensions and, accordingly, the weight of the device, etc. In the claimed design, it is possible to install a spring of almost any force without changing the moving part, the range of vertical movement can be easily increased by lengthening the "swing" arm from the side of the hand (the first and second levers of the device), and the increased weight is corrected by the counterweight on the drive part of the "swing". This allows the device to be used to work by moving the hand itself, as well as the hand with any heavy object, for example, a working tool, which greatly expands the scope of this device.

Thus, in the claimed device, spring (s) of constant force, for example, strip springs, with a constant pulling force are used as a compensating moment from the weight of the hand. The design is implemented according to the principle of a swing - on one side of which the patient's arm is located from the axis of rotation, on the other, the force created by the spring. The point of application of the spring force can move away or approach from the swing axis, thereby changing the amount of torque from the spring to compensate for different arm weights. In order for the arm support to perform plane-parallel movements when moving along the height, the structure uses a scheme with an articulated parallelogram (by analogy with the prototype), made with a length corresponding to the average size of a person's forearm, and fixed on a vertical stand. The location of the spring is chosen so that regardless of the angle of rotation within $\pm$ 30 degrees from the horizontal, the value of the arm of the force application does not change by more than 25% for each position of the load. The weight of the opposite parts of the "swing" is selected so as to balance the shoulders of the "swing" as much as possible without applying a load. A block with constant force springs is located as close as possible to the vertical axis of rotation of the device to reduce the moment of inertia from the entire device.

**Brief Description of Drawings**

The invention is illustrated by drawings and photographs. Figure 1 shows a photograph of a general view of the device; figure 2 is a side view of the device, which shows a cross-section of individual units / blocks; figure 3 and figure 4 - a diagram of the movement of the support for the hand in the vertical plane, and the change in the length of the arm of the application of the traction force of the springs when changing the angular position of the first lever of the device; figure 5 is a diagram of the possible positions of the levers of the device and the available areas for the hand; Figures 6-9 show individual units of the claimed device, showing possible variants of execution / connection of individual structural elements, namely, in Fig. 6 - assembly for connecting the pivot frame and the second lever of the device in a vertical section along the axis 16 (the axis 17 is similarly made, connecting the second lever 15 with the cradle 14); figure 7 is a spring of constant force in a horizontal section along the axis of the spring; Fig. 8 is a joint assembly of the first lever 6 of the pivot frame 4 with the drive bracket 7 in a horizontal section along axis 5 (the axis 13 of the connection of the first lever 6 with the side element 9 is similarly made); in fig. 9 - unit for connecting the body of the spring block 2 with the pivot frame 4 and the drive bracket 7, as well as with the vertical axis 3 of the support post 33.

Positions in the figures indicate:

1. - support post,

2. - the body of the spring block,

3. - vertical axis of the support post,

4. - pivot frame

5. - horizontal axis of frame fastening in body 2,

6. - the first lever of the pivot frame 4,

7. - the shoulder of the first lever 6, which acts as a drive bracket (drive bracket),

8. - rod of the pivot frame 4,

9. - side element of the pivot frame (which can be made in the form of a bracket),

10. - articulated connection of the rod 8 with the body 2, for example, in the form of a hinge tip,

11. - articulated connection of the rod 8 with the side element 9, for example, in the form of a hinge tip,

12. - articulated connection of the first lever 6 with the body 2, and with the side element 9,

13. - the horizontal axis of the connection of the first lever 6 with the side element 9,

14. - supporting element for the hand (cradle),

15. - the second lever,

16. - the vertical axis of the connection of the second lever 15 with the pivot frame 4,

17. - vertical axis of connection of the hand cradle with the second lever 15,

18. - horizontal axis of connection of the hand cradle with the second lever 15,

19. - tensioner block (adjustment of resistance to hand movement),

20. - an elastic element made in the form of a constant force spring,

21. - a bearing located in an elastic element 20,

22. - the axis of the bearing 21 for the elastic element 20,

23. - the screw of the tensioner block 19,

24. - screw handle 23,

25. - an element for fastening the end of the elastic element 20 to the screw 23, which can be made in the form of a nut,

26. - a groove made in the drive bracket 7 for placing the screw 23 and indicating the position of the end of the elastic element 20,

27. - limiter of angular movement of the pivot frame 4 in the vertical plane, fixed in the housing 2,

28. - angular contact bearings of all vertical axes and horizontal axes of the first lever,

29. - a clamp for fixing the device on a horizontal surface, for example, on a table or on a wheelchair,

30. - lock nut,

31. - wheel support,

32. - sleeve bearings located on the vertical axis 3,

33. - means for adjusting the support post with height,

34. - cap nut,

35. - washer,

36. - bushing,

37. - lock nut,

38. - screw,

39. - retaining ring,

40. - decorative nut,

41. - threaded coupler.

**Implementation of the invention**

The claimed invention is a device for supporting or supporting a hand and provides dynamic resistance to its movement (up or down), regardless of the position of the hand relative to the body (by compensating for the forces of gravity). The device is equipped with a unit for adjusting the resistance to hand movement with the possibility of individual adjustment for each patient, as well as a height-adjustable stand that allows the device to be positioned in a place convenient for the patient, at a convenient height.

Below is a more detailed description of the claimed invention, which does not limit the essence presented in the independent claim, but only demonstrates the possibility of achieving the claimed technical result.

The present invention may be subject to various changes and modifications that will be understood by a person skilled in the art based on reading the present description. Such changes do not limit the scope of the claim. For example, various means of connecting structural elements of the device, known from the prior art, can be used, which provide the ability to move / rotate individual parts of the device; means for adjusting the height of the device; means for mounting and / or securing the device to the supporting surface; materials for the execution of individual parts; any number of tensioner block springs (or spring block) can be used, interconnected to create the required force; the cradle for placing the hand can have a different constructive implementation; the device can be equipped with an electric or hydraulic drive for changing its height, as well as a means for adjusting the arm of the force application from an elastic element, which can also be performed using stepper motors, linear actuators, etc. Based on the specific tasks and scope of the device, it can be equipped with various additional accessories, sensors and tools for adjusting individual blocks and structural elements. The device can be implemented in whole or in part in a mirror-symmetrical design relative to the horizontal plane.

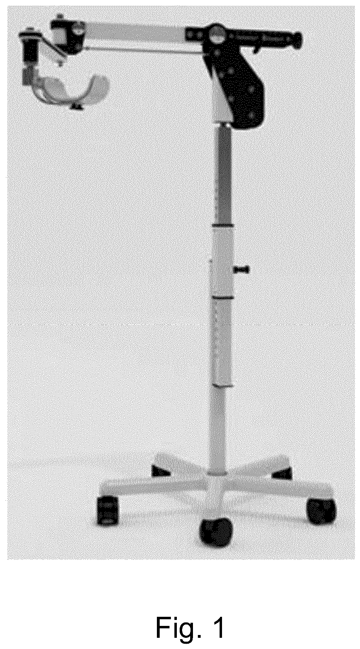

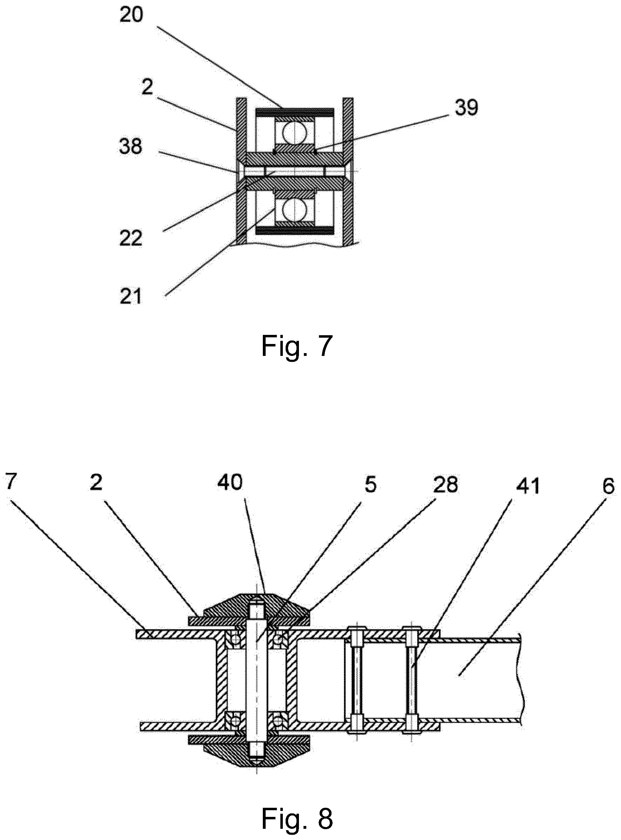

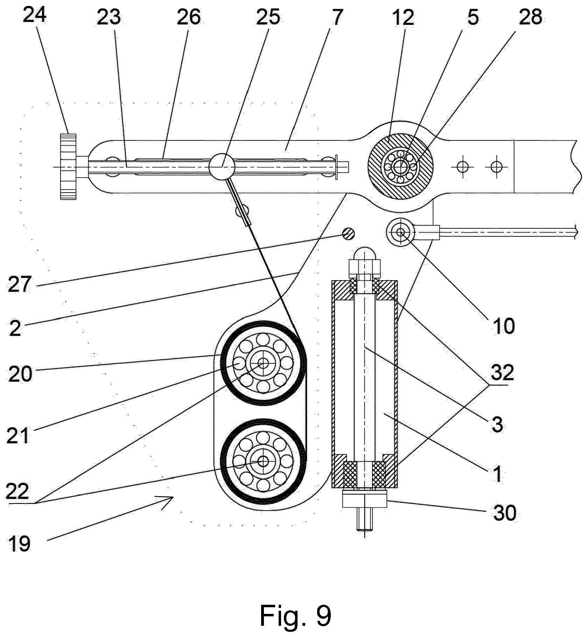

Below is a detailed description of the implementation of the invention on the example of a specific implementation of individual parts and assemblies (see figure 1 - 9).

The device is placed on a vertical support 1, which in one embodiment of the invention can be fixed on a free-standing base with a wheel support 31. In another embodiment of the invention, the support post 1 can be fixed, for example, with a clamp 29 on the working surface ( e.g. on a tabletop). In one embodiment of the invention, the vertical post can be made of two parts that move relative to each other (for example, in the form of a telescopic mechanism) with the possibility of fixing the selected position using a cylindrical pin installed in the holes made in the walls of the individual parts of the post.

The device includes, fixed on a post, for example, by means of a body 2, a pivot frame 4, functionally connected to a cradle 14 for placing a hand; tensioner block 19, including elastic elements 20 (spring block), functionally associated with elements for adjusting the resistance to hand movement, located on the shoulder 7 (drive bracket), which is a continuation of the first lever 6 of the pivot frame 4.

The body 2 is designed for the safe placement of springs in it, and is also the basis for fixing individual structural blocks and elements on it. In this regard, the housing can have a different design solution that provides the above-mentioned functions.

The pivot frame 4 is made with the possibility of rotation around the vertical axis 3 and angular movement in the vertical plane relative to the horizontal axis 5. The rotation of the pivot frame 4 about the vertical axis 3 can be realized by fixing the frame in the body 2, which in turn is fixed on a bushing having a threaded axis 3 with sleeve bearings 32, which is connected to the support post 1 through a lock nut 30. The angular movement of the pivot frame 4 in the vertical plane relative to the horizontal axis 5 can be realized by placing said axis directly on the first lever 6 of the pivot frame and fixing it in the body 2 on a —angular contact bearings 28.

The spring unit in one of the embodiments of the invention includes two springs of constant force 20, placed in the body 2. As springs of constant force, springs of the KKF series can be used (http://www.itkstr.ru/assets/download/spiralnyie.pdf) or similar. Springs 20 are mounted on bearings 21 to reduce unwinding resistance, which in turn are mounted on axles 22 fixed in the body 2.

The pivot frame is made by analogy with the prototype, includes upper and lower elongated elements - the first lever 6 and the rod 8, respectively, pivotally connected to the lateral internal and external elements to form a parallelogram. In this case, the body 2 acts as a side inner element, and element 9, which can be made in the form of a bracket, acts as a side outer element. The outer side element 9 is made with the ability to move vertically (up and down) relative to the inner side element while maintaining the parallel arrangement of the mentioned elements.

A feature of the claimed invention is the implementation of the pivot frame 4 with the shoulder 7 (drive bracket), which is connected to the first lever 6 of the pivot frame 4, and, in fact, is its continuation, as well as the implementation of the tensioner block 19, which is located on the side opposite to the placement of the cradle with respect to support, and includes a spring block located in the body 2 and separate structural elements for adjusting the spring force to provide the required resistance to hand movement - a groove 26 with a screw 23 placed in it, equipped with a handle 24 and an element 25 that secures the end of the elastic element 20 to the screw 23.

The shoulder 7 (drive bracket) of the pivot frame 4 is preferably made of carbon steel to compensate for the weight of the opposite part of the device, the pivot frame 4, connected to the second lever 15 and the cradle 14, preferably made of light aluminum alloys. The drive bracket 7 is provided with an axial groove 26 with a steel screw 23 with a trapezoidal thread placed therein to facilitate and accelerate the adjustment of the compensating force. The screw 23 is equipped with a nut 25, made with the possibility of moving along the screw, while the nut is equipped with a fastening of springs 20. The nut 25 moves when the handle 24 located at the end of the screw 23 rotates. The range of nut movement is selected to provide a threefold change in the compensating force. In one of the embodiments of the invention, the initial position of the groove with the screw is located at a distance of no more than 65 mm from the axis 5, the final position is at least 205 mm. This provides a threefold change in the value of the compensating force, sufficient to cover the required adjustment range, with the minimum dimensions of the device with the selected force. Changing the shoulder of application of force three times allows you to change the magnitude of the torque three times, $M = F \times L$, where F is the total force of the springs of constant force, for an adult (the most suitable value is 120N for the selected scheme), L is the shoulder of application of force, variable in the range of 65-205mm for an adult hand. Attached to the drive shoulder 7 is the first lever 6, which is a continuation of the drive shoulder 7. If the length of the first lever 6 is constant, when the torque value changes three times, the force at the end of the said first lever also changes three times. The force from 2 to 6 kg covers almost completely the required range of values for the weight of an adult hand. When the length of the first lever is 400mm, two springs with a force of 6kg can be used. The design also provides for the possibility of changing the nominal value of the compensated force by changing the number or force of the springs. By installing one spring instead of two, the device can be used for child rehabilitation. With an increase in the force of the springs, for example, by using two springs of 10 kg each or in a combination of 10 and 6 kg, the structure can be used to solve special problems, for example, to compensate for the weight of the hand together with the tool, which is relevant when using the inventive device, for example, when carrying out restoration work or any other work related to long-term holding of the hand in a certain position.

A second lever 15 is attached to the outer side element 9 of the pivot frame 4 in continuation of the drive bracket 7, connected to the first lever 6 through the vertical and horizontal axes of rotation, attached to the side element 9, which can be made in the form of a bracket. In this case, the horizontal axis of rotation 13 can be placed in the articulated connection 12, and the vertical axis 16 - in the bushing fixed on the said bracket 9. The second end of the rod 8 is also fixed on the bracket 9 (the first end of the rod is fixed in the body 2).

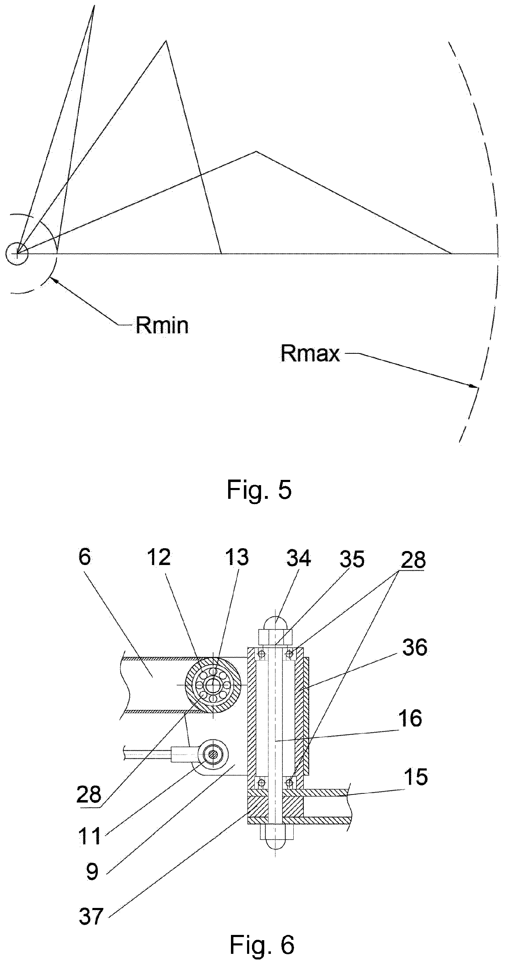

A rod 8 with two articulated ends 10 and 11 is used to maintain a horizontal position of the arm support. To limit the angle of rotation, a limiter 27 is used, which can be made in the form of a jumper welded into the body 2. The angle of rotation of the pivot frame, namely the first lever of the pivot frame, is set ± 30 ° from its horizontal position (Fig. 5). In this angular range, the movement along the radius of the arc is not much different from the rectilinear movement and allows you to comfortably use the support of the hand. Next to the horizontal axis 13 is the vertical axis of rotation 16 of the second lever 15. To maintain the position of the hand in space when turning around the axis 16, the axis 17 is intended. The distance between the axes 5 and 13 is preferably equal to the distance between the axes 16 and 17, so that the hand can be brought close to support post 1 (or vertical axis 3). Cradle 14 serves to accommodate the hand with its fixation, for example, using removable straps. All axles 5, 13, 16 and 17 are mounted with angular contact bearings to reduce friction during rotation.

Elements of the device: limiter 27, first lever 6, rod 8, bracket 9 along axes 5, 13 and points of attachment of rod 8 in hinge ends 10 and 11 form a parallelogram, which ensures the vertical position of axis 16 in the bracket when the swing frame 4 moves up or down 9, and, accordingly, the horizontal position of the second lever 15. The movable elements are connected by means of axes 5, 13, 16 and 17, which are threaded at the ends for clamping and selection of clearances in angular contact bearings. The housings of the axles 16 and 17 can be made in the form of bushings fixed at opposite ends of the second arm made of an aluminum alloy, preferably of a closed profile. The second lever 15 on the one hand is connected by the axis 16 with the bracket 9, and on the other hand by the vertical axis 17 with the cradle 14. In the lower part, the axle 17 is connected by a hinge to the horizontal axis 18, around which the cradle 14 (for the forearm) rotates. The presence of two mutually perpendicular axes allows you to move the cradle 14 (support for the hand) in space in any direction. The position of the patient's hand in space can be changed by the action of the hand through the cradle 14, the hinge with the horizontal axis 18, the vertical axis 17, the second lever 15, the bracket 9, on the first lever 6, which can rotate around the axis 5 in the vertical plane and axis 3 in the horizontal plane.

The claimed design of the arm support device allows free movement within a volume limited by the maximum radius and height of movement. Thanks to the three vertical axes 3, 16 and 17 with free rotation, the arm support - cradle 14, can be brought to any point in the area limited by the maximum radius equal to the extended length of the arm support (the total length of the first and second levers) and the minimum radius - the size of the post. In height, the cradle 14 moves due to rotation around the axis 5 and is held in a horizontal position due to the presence of rod 8. The amount of movement of the hand in height vertically H + H is equal to the distance between the axes of rotation 5 and 13 due to the rotation angles ± 30 degrees.

The claimed design realizes the ability to compensate for the weight of the hand with the most uniform effort during movement. One or more constant force springs are used as the compensating force. The spring creates a torque that is transformed into a supporting force in the A / 2H ratio based on the equality of the moments, and therefore, the force of the springs and the amount of movement can be selected depending on the tasks. Springs can be paired so that a range of forces can be matched by adding forces or using one spring instead of two. The arrangement of the springs, namely, the angle Y tangentially to the spring between the extreme positions of the adjustment, is selected so as to minimize the oscillations of the arm of the application of the force L depending on the angle of rotation: Lhoriz / Lup or Lhoriz / Ldown does not exceed 25%. In this case, the force is adjusted when the point of application of the force is moved along the groove 26. The movement is carried out by rotating the handle 24.

When using the device, the patient, in a comfortable position for him, places his forearm on the arm support - in the cradle 14. If necessary, the arm can be additionally fixed on the support, for example, using a flexible strap, etc. The patient chooses the height of the arm in space, the bending angles in the shoulder and elbow joints. In this position, the height of the rack is fixed and the device is balanced using the springs 20 and the mounting position for the springs 25 using the handle 24. In the equilibrium state, the moment of force - the force of gravity of the hand, on the shoulder of the first lever, remote from the center of the axis 5, should be approximately equal the force of action of the springs 20 applied to the arm, determined by the distance from the location of the attachment for the springs 25 to the center of the axis 5. This does not always require an exact compensation of the said forces. For example, to solve certain problems related to training the muscles of the arm, using the tensioner block, the force of the springs can be regulated to provide additional resistance when moving the arm. Equilibrium state - the force of gravity from the hand on the shoulder from axis 5 to axis 13 plus the moment of rotation from the right side of the device (constant value) should be equal to the moment from the left side of the device (constant value) plus the moment from the springs - the product of the height lowered from axis 5 by the line of tension of the spring from point 25 tangentially to the spring, namely:

$$F_n * L_{pr} + M_{priv} = M_{pod} + mg * L_1,$$

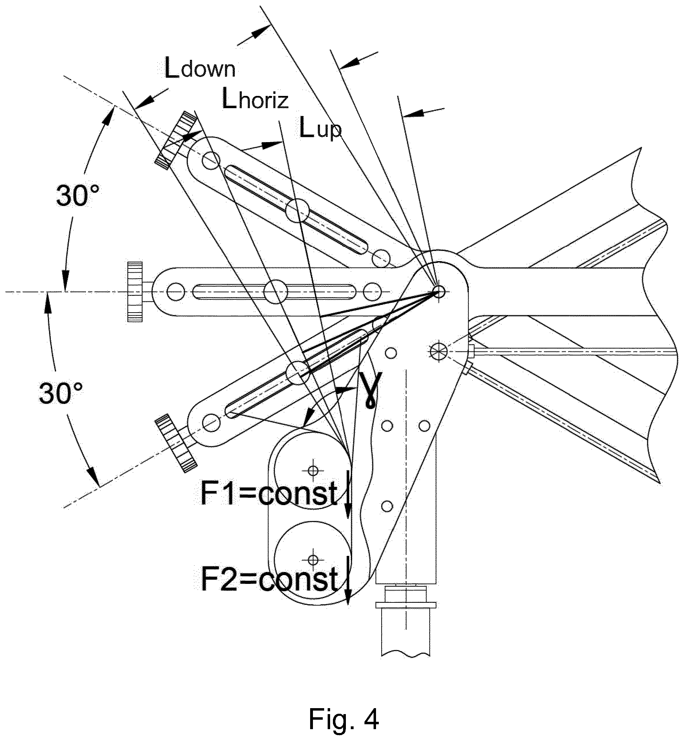

where: $F_n$ is the total force from the springs of constant force from 1 to n pieces, $L_{pr}$ is the perpendicular drawn from the center of rotation of the axis 5 to the spring, corresponds to $L_{down}$, $L_{horiz}$, $L_{up}$ in Fig. 4, $M_{priv}$ is the torque from the weight of the drive part of the arm support (constant value), $M_{pod}$ - moment of rotation from the supporting part of the device (constant value), mg - hand gravity, $L_1$ - distance between axes 5 and 13 (constant value).

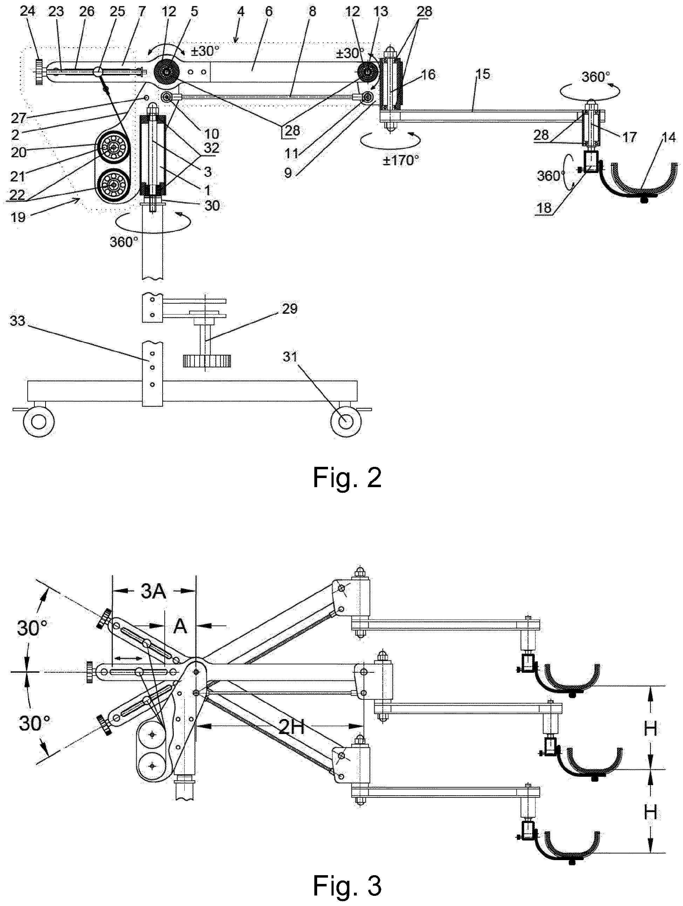

After balancing the device, the patient can change the position of the hand along any path in the zone accessible and determined by the dimensions of the levers 6 and 15 of the device. The area available for angular movement is mainly determined by the angle of possible movement of the first lever 6 relative to the axis 5 - in one embodiment, +/- 30 degrees relative to the horizontal (Fig. 3.4).

An example of implementation of the invention.

A device was made to support the arm (see figure 1), in which two springs of constant force of 60N were used; the distance between the axis of rotation 5 and 13 was 400 mm, the distance between the axes 16 and 17 was 400 mm, the angle of rotation of the first lever was ± 30 degrees, the adjustment range of the point of application of the force of the springs was 65—205 mm from the axis of rotation of position 5, the axis of rotation of the upper constant spring force was located at a distance of 150 mm vertically and 105 mm horizontally from the axis of rotation 5, the drive bracket was made of steel with a density of 7850 kg/m$^3$, the first and second levers were made of aluminum alloys with a density of 2730 kg/m$^3$.

As test shows, weights weighing 2 kg, 3 kg, 4 kg, 5 kg, 6 kg were suspended from the hand cradle. By adjusting the tensioner block, the load was balanced in a horizontal position. Next, the cradle was moved to extreme positions using a dynamometer, measuring the maximum effort. In the entire measuring range, the force did not exceed 0.5 kg. Most of the travel range does not exceed 0.15kg. In this connection, the maximum possible constant force of support of the hand was provided in the entire range of hand movement with a minimum inertia of the device.

Thus, the claimed design allows, with minimal resistance and inertia, to perform natural hand movements in full, including (flexion / extension) in the elbow and wrist joints with the involvement of the muscles of the proximal shoulder, abduction of the shoulder in the frontal plane, and precise adjustment of the compensating force. Due to the small deviation of the compensating force from the set value in the entire range of motion, it allows working with patients with very complex injuries from the very beginning of rehabilitation. Due to the design with different forces, the device is suitable for medical rehabilitation of children and adults, as well as the possibility of using the device in other areas as a support for the hand during various works.

## Claims

1. An arm support device, comprising:

   - a pivot frame connected to a support post with the ability to rotate around a vertical axis of the support post and with the ability of an angular movement relative to a horizontal axis of the pivot frame in a vertical plane, while the pivot frame includes a pivotally connected upper element in the form of a first lever, a lower element in the form of a rod and side elements forming a parallelogram,
   - a support element for a hand, functionally connected to the pivot frame through a second lever, wherein the second lever is rotatable relative to the vertical axis of the connection of the second lever with the pivot frame,
   - a tensioner unit, including an elastic element connected to the pivot frame with the ability to adjust a tension force of the elastic element to compensate for a weight of the hand;

   wherein the first lever of the pivot frame is made with a shoulder in the form of a drive bracket, the tensioner unit is located on the opposite side of the pivot frame relative to the axis of the support post; wherein at least one constant force spring is used as the elastic element of the tensioner unit, the free end of said constant force spring is connected to the drive bracket.

2. The device according to claim 1, wherein the connection of the pivot frame with the support post is made by means of a body fixed to the support post with the possibility of rotation of said body in the horizontal plane relative to the vertical axis of the support post, and the pivot frame is fixed in the body with the possibility of angular movement of said body relative to the horizontal axis for fastening the frame to the body.

3. The device according to claim 1, wherein the tensioner unit is fixed on the body.

4. The device according to claim 1, wherein the body is made in the form of two flat metal plates between which spring elements are fixed on the horizontal axes.

5. The device according to claim 1, wherein 2 springs of the KKF series with a total force of at least 120N are used as constant force springs.

6. The device according to claim 1, wherein the drive bracket is made of steel, and the first and second levers are made of aluminum alloys.

7. The device according to claim 1, wherein in order to adjust the tension of the elastic element, the tensioner unit is equipped with a screw located in the drive bracket, made with a handle and an element for fastening the end of the elastic element with the possibility of moving along the screw and fixing in a selected position, while the drive bracket is provided with a corresponding groove for placing the screw with said element, as well as a scale for controlling the compensating force.

8. The device according to claim 7, wherein the length of the groove on the drive bracket with the screw located therein is selected to provide a threefold change in the compensating force.

9. The device according to claim 7, wherein an initial position of the groove with the screw placed therein is selected at a distance of no more than 65 mm from the horizontal axis of the first lever attachment to the body, and a final position is not less than 205 mm.

10. The device according to claim 1, wherein the first lever is made with a length from 350 mm to 450 mm, and the tensioner block includes two elastic elements in the form of constant force springs with a total force of up to 12 kg.

11. The device according to claim 2, wherein the movement of the frame relative to the horizontal axis of fastening the frame to the body is limited by an angle of rotation of $\pm$ 30 ° from the horizontal position by means of a limiter fixed on the body.

12. The device according to claim 1, wherein the side element of the frame is made in the form of a bracket on which the vertical axis of the connection of the second lever with the pivot frame is fixed.

13. The device according to claim 1, wherein the first and second levers are made with a length that allows the hand support element to be brought to the support post.

14. The device according to claim 1, wherein the support element for the hand is rotatable around the vertical and horizontal axes, by means of which the connection of the support element with the second lever is made.

15. A device according to claim 1, wherein the hand support element is provided with removable straps.

16. The device according to claim 1, wherein the vertical axes and the horizontal axes of the first lever are installed in angular contact bearings, and the axes at the ends have a threaded section for adjusting the size of the clearances in the angular contact bearings.

17. The device according to claim 1, wherein the second lever is made with a closed cross-sectional profile.

18. The device according to claim 1, wherein the support post is designed to be mounted on a horizontal working surface or on a movable base.

19. The device according to claim 1, wherein the drive bracket is made in the form of an extension of the first lever.

20. The device according to claim 1, wherein the support post is made with the possibility of changing the height with the fixation of the selected position.

Fig. 1

Fig. 2

Fig. 3

Fig. 4

Fig. 5

Fig. 6

Fig. 7

Fig. 8

Fig. 9

## INTERNATIONAL SEARCH REPORT

| International application No. |
| --- |
| PCT/RU 2019/000081 |

**A. CLASSIFICATION OF SUBJECT MATTER**

A61F 5/37 (2006.01); A61H 1/02 (2006.01); A61G 15/12 (2006.01)

According to International Patent Classification (IPC) or to both national classification and IPC

**B. FIELDS SEARCHED**

Minimum documentation searched (classification system followed by classification symbols)

A61F5/37, A61H 1/02, A61G 15/12

Documentation searched other than minimum documentation to the extent that such documents are included in the fields searched

Electronic data base consulted during the international search (name of data base and, where practicable, search terms used)

Espacenet, PatSearch (RUPTO Internal), USPTO

**C. DOCUMENTS CONSIDERED TO BE RELEVANT**

| Category* | Citation of document, with indication, where appropriate, of the relevant passages | Relevant to claim No. |
| --- | --- | --- |
| D, A | US 2013/0087154 A1 (SAEBO, INC.) 11.04.2013, the abstract, fig.1, 5 | 1-20 |
| D, A | RU 166673 U1 (OBSCHESTVO S OGRANICHENNOI OTVETSTVENNOSTJU «LABORATORIYA VYSOKOTOCHNOI MEKHANIKI I OPTIKI») 10.12.2016 | 1-20 |
| A | US 6695795 B2 (GERD KNOLL) 24.02.2004 | 1-20 |
| A | DE 10101214 A1 (OPED AG) 22.08.2002 | 1-20 |

☐ Further documents are listed in the continuation of Box C.  ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 30 September 2019 (30.09.2019) | 31 October 2019 (31.10.2019) |

| Name and mailing address of the ISA/ RU | Authorized officer |
| --- | --- |
| Facsimile No. | Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106901945
- RU 166673

- US 2013087154 A